(19) Europäisches Patentamt
European Patent Office
Office européen des brevets

(11) **EP 1 710 575 B1**

(12) **FASCICULE DE BREVET EUROPEEN**

(45) Date de publication et mention
de la délivrance du brevet:
**08.12.2010 Bulletin 2010/49**

(51) Int Cl.:
***G01N 33/24*** *(2006.01)*

(21) Numéro de dépôt: **06290504.7**

(22) Date de dépôt: **30.03.2006**

(54) **Procédé de détermination de la teneur en au mois un gaz donné dans une boue de forage, dispositif et installation associes**

Verfahren zur Bestimmung des monatlichen Gehalts mindestens eines gegebenen Gases in einem Bohrschlamm, zugehörige Vorrichtung und Anlage

Procedure for determining the monthly content of a given gas in drilling mud and associated device and installation

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **04.04.2005 FR 0503317**

(43) Date de publication de la demande:
**11.10.2006 Bulletin 2006/41**

(73) Titulaire: **Geoservices Equipements**
**93150 Le Blanc-Mesnil (FR)**

(72) Inventeurs:
• **Frechin, Nicolas**
**75019 Paris (FR)**
• **Breviere, Jérome**
**95150 Taverny (FR)**

(74) Mandataire: **Domenego, Bertrand et al**
**Cabinet Lavoix**
**2, place d'Estienne d'Orves**
**75441 Paris Cedex 09 (FR)**

(56) Documents cités:
**FR-A1- 2 773 219     US-A- 5 049 738**
**US-A- 5 375 465     US-A- 5 859 430**

**Description**

La présente invention concerne un procédé de détermination de la teneur en au moins un gaz donné dans une boue de forage.

Lors du forage d'un puits de pétrole ou d'un autre effluent (notamment gaz, vapeur, eau), il est connu de réaliser une analyse des composés gazeux contenus dans les boues de forage émergeant du puits. Cette analyse permet de reconstituer la succession géologique des formations traversées lors du forage et intervient dans la détermination des possibilités d'exploitation des gisements de fluides rencontrés.

Cette analyse, réalisée en continu, comprend deux phases principales. La première phase consiste à extraire les gaz véhiculés par la boue (par exemple des composés hydrocarbonés, dioxyde de carbone, sulfure d'hydrogène). La deuxième phase consiste à qualifier et quantifier les gaz extraits.

Pour l'extraction des gaz de la boue, un dégazeur à agitation mécanique du type décrit dans FR 2 799 790 est fréquemment utilisé. Les gaz extraits de la boue, mélangés avec un gaz vecteur introduit dans le dégazeur, sont convoyés par aspiration à travers une conduite d'extraction de gaz jusqu'à un analyseur qui permet la quantification des gaz extraits.

Un tel procédé ne donne pas entière satisfaction, notamment pour les boues à base d'huile ou de produits synthétiques. En effet, la cinétique d'extraction des gaz dans le dégazeur dépend notamment de la solubilité des gaz dans la boue. Par exemple, pour une boue de forage pétrolier, les gaz d'hydrocarbures en $C_4$ sont extraits plus lentement que les gaz d'hydrocarbures en $C_1$.

Compte tenu du temps limité de résidence de la boue dans le dégazeur, la détermination de la teneur relative ou absolue des gaz contenus dans la boue de forage, à partir des quantités de gaz extraits dans le dégazeur requiert des modèles mathématiques complexes et manque de précision.

Par ailleurs, le document US 5,859,430 décrit un procédé de détermination de la teneur en plusieurs gaz de boue de forage comprenant une étape de mesure de l'absorption d'une radiation issue d'une source infrarouge par le (s) gaz contenu(s) dans la boue de forage, et une étape de correction du déplacement des pics d'absorption grâce à l'utilisation d'un échantillon de référence.

L'invention a donc pour but principal de déterminer de manière précise mais simple la teneur en au moins un gaz donné contenu dans une boue de forage, au cours d'un forage.

A cet effet, l'invention a pour objet un procédé selon la revendication 1.

Le procédé selon l'invention peut comporter une ou plusieurs des caractéristiques qui font l'objet des revendications 2 à 9.

L'invention a en outre pour objet un dispositif selon la revendication 10.

Le dispositif selon l'invention peut comporter une ou plusieurs des caractéristiques qui font l'objet des revendications 11 à 14.

L'invention a également pour objet une installation de forage selon la revendication 15.

Des exemples de mise en oeuvre de l'invention vont maintenant être décrits en regard des dessins annexés, sur lesquels :

- la Figure 1 est une vue schématique en coupe verticale d'une installation de forage munie d'un premier dispositif selon l'invention ;
- la Figure 2 est un organigramme explicitant une étape de calibration dans un procédé selon l'invention ;
- la Figure 3 est un organigramme explicitant une étape d'analyse dans un procédé selon l'invention ;
- la Figure 4 est un graphe représentant une famille de courbes représentatives de l'extraction d'un gaz donné dans un dispositif selon l'invention ;
- la Figure 5 est une vue analogue à la Figure 4 pour deux gaz donnés distincts; et
- la Figure 6 est une vue analogue à la Figure 1, l'installation étant munie d'un deuxième dispositif selon l'invention,
- la Figure 7 est une vue analogue à la Figure 1, l'installation étant munie d'un troisième dispositif selon l'invention.

Un dispositif selon l'invention est utilisé par exemple dans une installation de forage d'un puits de production de pétrole.

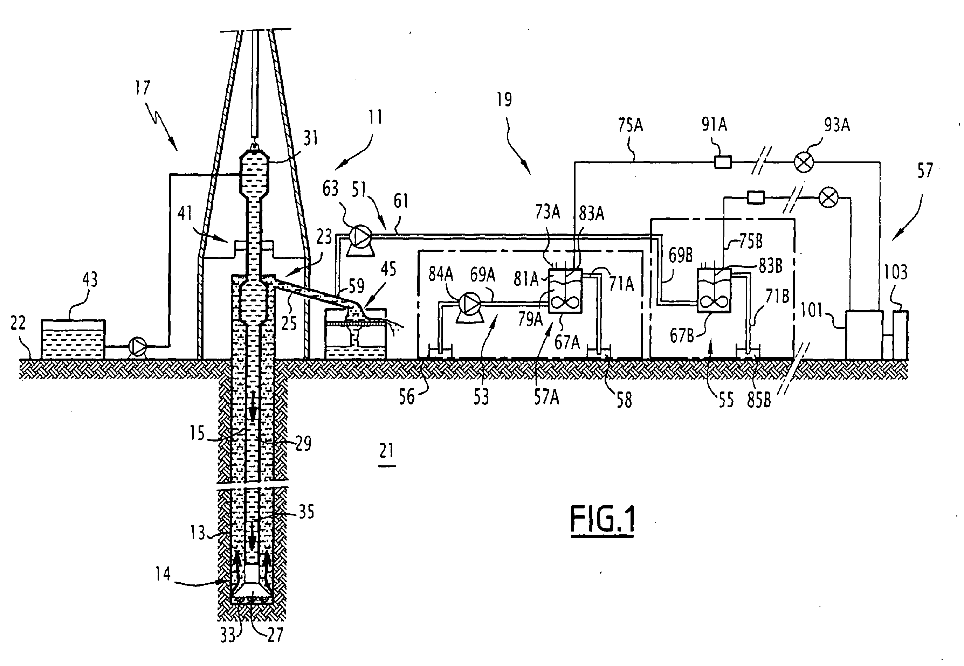

Comme illustré par la Figure 1, cette installation 11 comprend un conduit de forage 13 dans une cavité 14 percée par un outil de forage 15 rotatif, une installation de surface 17, et un dispositif 19 selon l'invention.

Le conduit de forage 13 est disposé dans la cavité 14 percée dans le sous-sol 21 par l'outil de forage 15 rotatif. Ce conduit 13 comporte, au niveau de la surface 22, une tête de puits 23 munie d'une conduite 25 de vidange.

L'outil de forage 15 comprend une tête de forage 27, une garniture de forage 29, et une tête 31 d'injection de liquide.

La tête de forage 27 comprend des moyens de perçage 33 des roches du sous-sol 21. Elle est montée sur la partie inférieure de la garniture de forage 29 et est positionnée dans le fond du conduit de forage 13.

La garniture 29 comprend un ensemble de tubes de forage creux. Ces tubes délimitent un espace interne 35 qui permet d'amener un liquide depuis la surface 22 jusqu'à la tête de forage 27. A cet effet, la tête d'injection 31 de liquide est vissée sur la partie supérieure de la garniture 29.

L'installation de surface 17 comprend des moyens 41 de support et d'entraînement en rotation de l'outil de forage 15, des moyens 43 d'injection du liquide de forage et un tamis vibrant 45.

Les moyens d'injection 43 sont reliés hydrauliquement à la tête d'injection 31 pour introduire et faire circuler un liquide dans l'espace interne 35 de la garniture de forage 29.

Le tamis vibrant 45 collecte le liquide chargé de résidus de forage, (désigné par la suite par « boue de forage ») qui sort de la conduite de vidange 25 et sépare le liquide des résidus de forage solides.

Comme illustré par la Figure 1, le dispositif 19 selon l'invention comprend un étage de calibration 53 et un étage d'analyse 55. Le dispositif comprend en outre des moyens 57 d'analyse et de calcul, communs à l'étage de calibration 53 et à l'étage d'analyse 55.

L'étage de calibration 53 comprend un bac 56 de réception d'un échantillon de boue de calibration, des moyens 57A d'extraction de gaz et un bac 58 de rétention des boues évacuées des moyens d'extraction.

Les moyens d'extraction 57 de gaz sont du type décrit dans la demande de brevet FR 2 799 790. Ces moyens comprennent une cuve 67A, une conduite 69A d'amenée de boue dans la cuve 67A, une conduite 71A d'évacuation de la boue de la cuve 67A, des moyens 73A d'introduction d'un gaz vecteur dans la cuve 67A, une conduite 75A d'extraction des gaz extraits dans la cuve 67A.

La cuve 67A comprend une partie inférieure 79A dans laquelle la boue circule et une partie supérieure 81A qui présente un ciel gazeux. Cette cuve 67A est par ailleurs munie d'un agitateur mécanique 83A.

La conduite d'amenée de boue 69A s'étend entre le bac 56 et la cuve 67A. Cette conduite d'amenée 69A est munie d'une pompe 84A de prélèvement et avantageusement de moyens de chauffage de la boue (non représentés), afin de porter la température de cette boue à des valeurs comprises entre 25 et 120°C, de préférence entre 60 et 90°C.

La conduite d'évacuation de boue 71A s'étend entre un passage à débordement, ménagé dans la partie supérieure 81A de la cuve 67A et le bac de rétention 58.

Les moyens d'introduction 73A d'un gaz vecteur dans la cuve 67A comprennent une prise d'air pour introduire de l'air à pression atmosphérique dans la cuve 67A.

La conduite d'extraction 75A s'étend entre une ouverture d'extraction ménagée dans la partie supérieure 81A de la cuve 67A et les moyens 57 d'analyse et de calcul.

La conduite d'extraction 75A comporte un régulateur de débit 91A et une pompe à vide 93A qui permet le convoyage par aspiration des gaz extraits de la cuve jusqu'aux moyens 57 d'analyse et de calcul.

L'étage d'analyse 55 comprend des moyens 51 de prélèvement de boue, des moyens 57B d'extraction des gaz, et un bac de rétention de boue 85B.

Les moyens de prélèvement 51 comprennent une tête 59 de prélèvement de liquide, montée en saillie dans la conduite de vidange 25, et une tubulure de raccordement 61.

La tubulure de raccordement 61 est munie d'une pompe 63 de prélèvement, par exemple une pompe péristaltique et s'étend entre la tête 59 et la cuve 67B des moyens de prélèvement.

Les moyens 57B d'extraction des gaz de l'étage d'analyse 55 sont de structure identique aux moyens d'extraction 57A de l'étage de calibration.

Ainsi, les cuves 67A et 67B des étages de calibration et d'analyse 53 et 55 sont de structure identiques, notamment par leur géométrie et par la nature de l'agitateur 83A et 83B.

Par ailleurs, la conduite d'extraction de gaz 75B de l'étage d'analyse 55 est reliée aux moyens 57 d'analyse.

La conduite 71 B d'évacuation de la boue de l'étage 55 est reliée directement à un bac de rétention 85B.

Les moyens d'analyse et de calcul 57 comprennent une instrumentation 101 et un calculateur 103.

L'instrumentation 101 est munie d'un collecteur de gaz sélectif qui reçoit sélectivement les gaz extraits par l'une ou l'autre des conduites d'extraction 75A ou 75B respectives de l'étage de calibration 53 et de l'étage d'analyse 55 et qui convoie ces gaz extraits jusqu'à au moins un appareil de mesure.

L'appareil de mesure est par exemple un appareil à détection infrarouge pour la quantification du dioxyde de carbone, un chromatographe FID (détecteur à ionisation de flammes) pour la détection des hydrocarbures ou encore TCD (détecteur à conductivité thermique), en fonction des gaz à analyser. La détection et la quantification simultanée d'une pluralité de gaz est donc possible, notamment si l'instrumentation 101 comprend plusieurs appareils de mesure.

Dans l'exemple représenté, au moins un appareil génère un signal électrique qui est fonction de la quantité de gaz donné dans les gaz extraits collectés et transmet ce signal au calculateur 103.

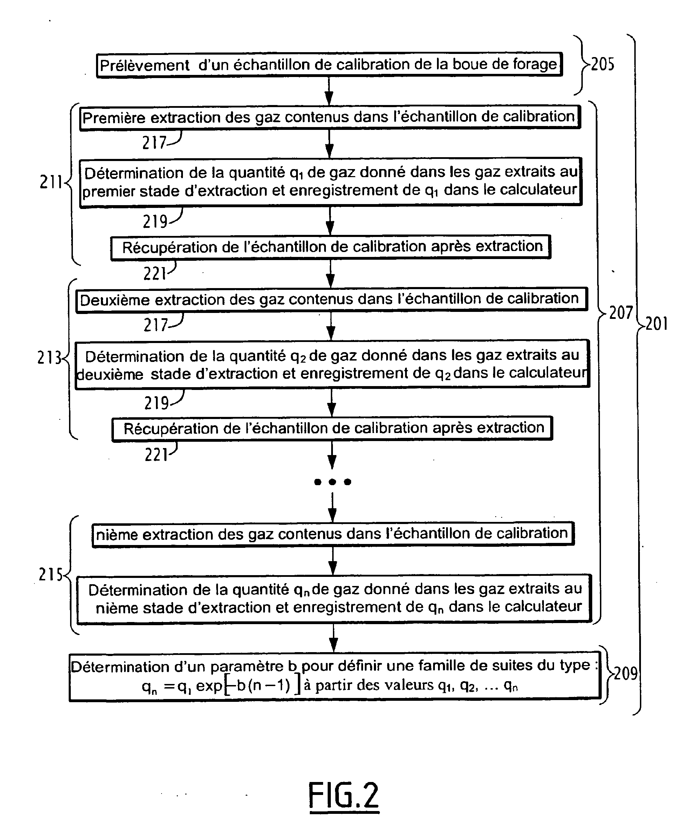

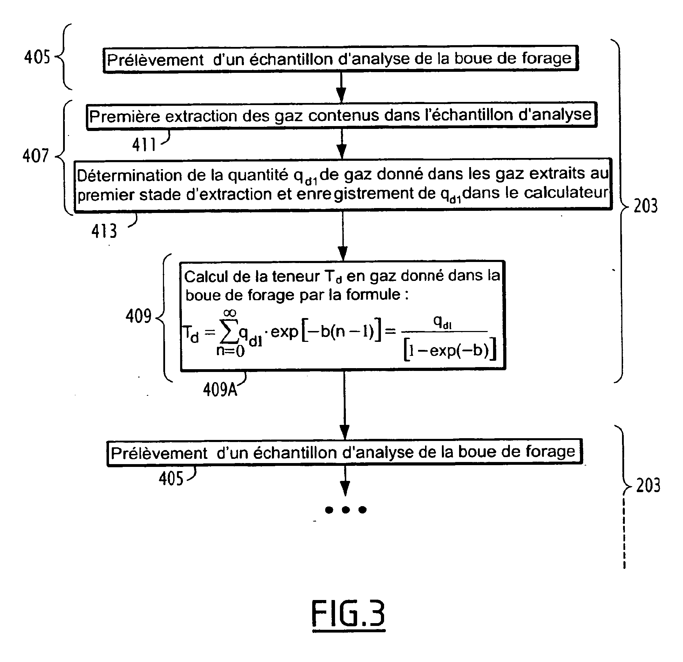

La mise en oeuvre du procédé selon l'invention, pour déterminer la teneur en au moins un gaz donné lors d'une phase de forage d'un puits va maintenant être décrit comme exemple, en référence à la Figure 1 et aux organigrammes des Figures 2 et 3.

Les gaz donnés analysés par ce procédé sont par exemple des hydrocarbures en $C_1$ à $C_6$, de préférence en $C_1$ à $C_5$.

Lors de la phase de forage, l'outil de forage 15 est entraîné en rotation par l'installation de surface 41. Un liquide de forage est introduit dans l'espace intérieur 35 de la garniture de forage 29 par les moyens d'injection 43. Ce liquide descend jusqu'à la tête de forage 27, et passe dans le conduit de forage 13 à travers la tête de forage 27. Ce liquide refroidit et lubrifie les moyens de perçage 33. Puis, le liquide collecte les déblais solides résultant du forage et remonte par l'espace annulaire défini entre la garniture de forage 29 et les parois du conduit de forage 13. La boue de forage ainsi formée est évacuée par la conduite de vidange 25.

Le terme « phase de forage » désigne ici l'opération de perçage du conduit de forage 13 dans une formation du sous-sol de nature sensiblement homogène. La composition du liquide de forage introduit dans l'espace 35 durant une phase de forage est sensiblement constante.

La boue de forage évacuée par la conduite 25 est par exemple une boue à base d'eau, une boue à base d'huile, ou une boue à base d'un matériau synthétique, en fonction de la nature du liquide de forage introduit dans l'installation 11.

Comme illustré par les Figures 2 et 3, le procédé selon l'invention comprend pour chaque phase de forage, au moins une étape de calibration 201 et une pluralité d'étapes d'analyse 203.

Le nombre d'étapes de calibration 201 est inférieur au nombre d'étapes d'analyse 203. De préférence, une étape de calibration 201 unique est effectuée pour l'ensemble des étapes d'analyse 203 lors d'une même phase de forage.

Comme représenté sur la Figure 2, l'étape de calibration 201 comprend successivement une phase de prélèvement 205, une phase de mesure 207 et une phase de calcul 209.

Dans la phase de prélèvement 205, un échantillon de calibration de la boue de forage qui circule dans la conduite de vidange 25 est recueilli dans le bac de réception 56. Cet échantillon a un volume prédéterminé par exemple supérieur à 150 fois le volume de la cuve 67A. De préférence ce volume est égal à environ 200 fois le volume de la cuve 67A.

La pompe 84A de l'étage de calibration 53 est activée de sorte que l'échantillon de calibration est convoyé jusqu'à la cuve 67A de l'étage de calibration 53 à travers la conduite d'amenée de boue 69A.

La phase de mesure 207 comporte au moins deux stades d'extraction 211, 213, 215 successifs effectués sur le même échantillon de calibration.

Dans un premier stade d'extraction 211, l'échantillon de calibration contenant une teneur initiale $T_0$ en gaz donné est introduit dans la cuve 67A, par exemple à un débit de 0,3 litres par minute. Dans la cuve 67A, une partie des gaz contenus dans l'échantillon de calibration est extraite de cet échantillon et collectée à l'étape 217 dans la partie supérieure 81A dans des conditions déterminées, notamment de température, de temps de résidence et de vitesse d'agitation. Dans l'exemple représenté sur la Figure 1, la température de l'échantillon est sensiblement égale à 90°C et le temps de résidence dans la cuve est de 16 secondes environ.

Une partie $q_1$ du gaz donné contenu initialement dans l'échantillon de calibration est donc extraite de cet échantillon dans le ciel gazeux de la cuve 67A.

Les gaz extraits sont prélevés par aspiration via la conduite d'extraction 75A et convoyés jusqu'à l'instrumentation 101 à travers cette conduite 75A, via le débitmètre 91A et la pompe à vide 93A. Le débit d'aspiration est par exemple sensiblement égal à 500 cm$^3$/min.

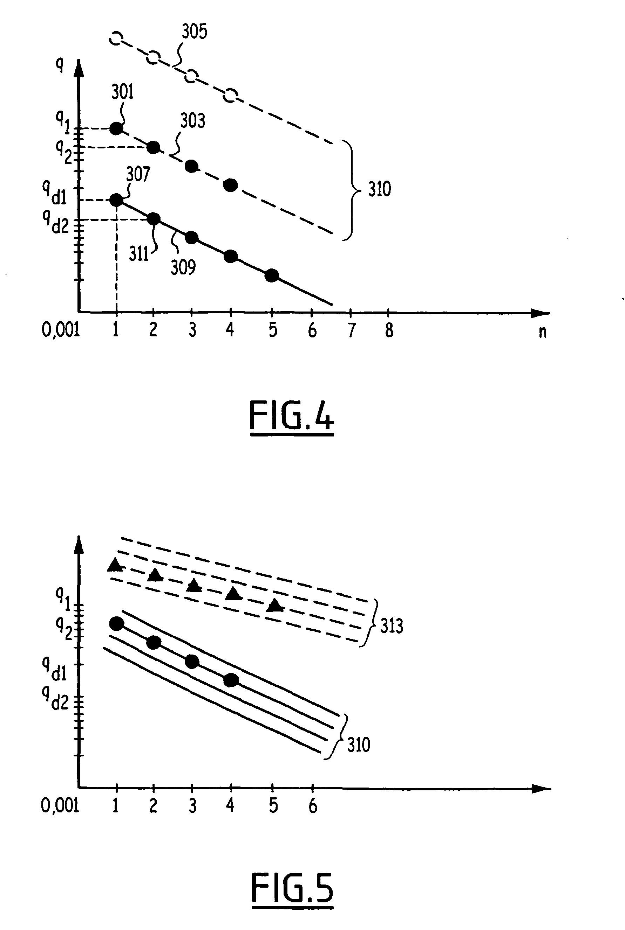

L'instrumentation 101 qualifie alors les gaz extraits de la cuve et génère un signal représentatif de la quantité $q_1$ de gaz donné dans ces gaz extraits. Cette quantité $q_1$ est alors enregistrée dans le calculateur 103 à l'étape 219 et associée par le calculateur 103 à un nombre entier qui correspond au stade d'extraction en cours (point 301 sur la Figure 4).

Puis, sensiblement la totalité de l'échantillon de calibration est récupéré dans le bac de rétention 58.

Le bac de rétention 58 et le bac de réception 56 sont alors permutés. Le bac 58 contenant la totalité de l'échantillon de calibration récupéré après son passage dans la cuve 67A est ainsi raccordé à la conduite de prélèvement 69A.

Le même échantillon de calibration, après avoir subi le premier stade d'extraction est donc récupéré à l'étape 221 et de nouveau introduit en totalité dans la cuve 67A pour y subir un deuxième stade d'extraction 213.

La teneur $T_1$ en gaz donné contenu dans l'échantillon de calibration avant le deuxième stade d'extraction est égale sensiblement à la différence entre la teneur initiale $T_0$ en gaz donné dans la boue et la quantité de gaz $q_1$ extraite de la boue lors du premier stade d'extraction.

L'échantillon de calibration subit alors un deuxième stade d'extraction 213, qui comprend les mêmes étapes 217, 219, 221 que le premier stade d'extraction 211.

Une quantité $q_2$ de gaz donné est extraite de la boue lors du deuxième stade d'extraction 213.

Pour mettre en oeuvre le procédé selon l'invention, au moins deux stades d'extraction 211, 213, 215 sont nécessaires. Dans l'exemple illustré, le nombre de stades d'extraction est compris entre 2 et 6.

Dans la phase de calcul 209, le calculateur 103 détermine la définition d'une suite représentée, en échelle logarithmique, par la courbe 303 de la Figure 4, à partir d'au moins deux couples de valeurs $(n, q_n)$ qui correspondent à deux stades d'extraction n des gaz de l'échantillon de calibration et à la quantité de gaz donné $q_n$ dans les gaz extraits lors des stades d'extraction n. Cette suite dépend de la quantité $q_1$ de gaz donné extraite lors d'un premier stade d'extraction 211 et d'un paramètre b caractéristique de la boue.

Comme illustré par la Figure 4, la suite représentée par la courbe 303 est sensiblement une suite géométrique exponentielle qui est décrite avantageusement par la formule :

$$q_n = q_1 \exp[-b(n-1)] \qquad (1)$$

dans laquelle :

- n est le nième stade d'extraction ;
- $q_1$ est la quantité de gaz donné dans les gaz extraits lors du premier stade d'extraction ;
- $q_n$ est la quantité de gaz donné dans les gaz extraits lors du nième stade d'extraction ; et
- b est un paramètre indépendant de la teneur $T_0$ initiale en gaz donné dans l'échantillon de calibration.

Ainsi, le paramètre b dépend uniquement des caractéristiques de la boue, notamment de sa composition, et des conditions déterminées.

A cet effet, si l'étape de calibration 201 était effectuée avec un échantillon de calibration comportant initialement une teneur en gaz donné $T_{d0}$ différente, la phase de mesure 207 effectuée avec cet échantillon permettrait, lors de la phase de calcul 209, d'obtenir une suite représentée par une courbe 305 parallèle à la première courbe 303, en échelle logarithmique.

Une famille 310 de suites, représentées respectivement par les courbes 303, 305, 309 et représentative de l'extraction, dans les conditions déterminées, du gaz donné contenu dans la boue de forage est donc établie grâce à la détermination du paramètre b. Ces courbes sont décrites par la formule générale (1), dans lequel n et $q_1$ sont les paramètres de définition de la famille, pour l'exemple illustré par la Figure 1.

Comme représenté sur la Figure 3, chaque étape d'analyse comprend une phase de prélèvement 405, une phase de mesure 407 et une phase de calcul 409.

Dans la phase de prélèvement 405, la pompe de prélèvement 63 de l'étage d'analyse 55 est activée, afin de prélever un échantillon d'analyse de la boue de forage à partir de la conduite de vidange 25.

L'échantillon d'analyse est convoyé jusqu'à la cuve 67B, via la conduite d'amenée 69B de l'étage d'analyse.

L'étape de mesure 407 comporte un stade unique d'extraction 411.

Dans le stade d'extraction, l'échantillon d'analyse contient initialement une teneur $T_d$ à déterminer en gaz donné. Cet échantillon est introduit dans la cuve 67B de l'étage d'analyse 55 où les conditions déterminées utilisées dans la cuve 67A de l'étage de calibration 53 durant l'étape de calibration (notamment température, temps de résidence et vitesse d'agitation) sont reproduites.

Les gaz extraits de l'échantillon d'analyse sont prélevés via la conduite d'extraction 75B et sont convoyés jusqu'aux moyens d'analyse et de calcul 57.

Un signal représentatif de la quantité $q_{d1}$ de gaz donné dans les gaz extraits de l'échantillon d'analyse lors de ce stade unique d'extraction est alors généré par l'instrumentation 101 et la quantité $q_{d1}$ est enregistrée par le calculateur 103 (étape 413) et associée au premier stade d'extraction (point 307 sur la Figure 4).

Dans la phase de calcul 409A, le calculateur 103 identifie la courbe 309 de la famille 310 de suites sur laquelle se trouve le point 307 correspondant à la quantité $q_{d1}$ de gaz donné dans les gaz extraits de l'échantillon d'analyse et au stade d'extraction dans laquelle la quantité $q_{d1}$ de gaz donné a été recueillie. Dans l'exemple illustré, cette courbe 309 passe par le point 307 d'intersection entre la parallèle à l'axe des abscisses, passant par la valeur $q_{d1}$ de l'axe des ordonnées, et la parallèle à l'axe des ordonnées passant par le point 1 de l'axe des abscisses, qui correspond au premier stade d'extraction dans la cuve 67.

Le calculateur 103 calcule alors la teneur initiale en gaz donné $T_{d0}$ dans l'échantillon d'analyse en effectuant la somme de la quantité de gaz donnée $q_{d1}$ dans les gaz extraits de l'échantillon d'analyse, avec la quantité de gaz donné $q_{d2}$ correspondant à au moins un autre point 311 de la courbe identifiée.

Dans l'étape de calcul 409A, la teneur $T_d$ est calculée par la formule :

$$T_d = \sum_{n=1}^{\infty} q_{d1} \cdot \exp[-b(n-1)] = \frac{q_{d1}}{[1-\exp(-b)]} \quad (2),$$

où $q_{d1}$ est la quantité de gaz donné dans les gaz extraits de l'échantillon d'analyse et b est le paramètre déterminé à l'étape de calibration.

En variante (non représentée), la teneur initiale en gaz donné est calculée par la formule :

$$T_d = \sum_{n=1}^{N} q_{d1} \exp[-b(n-1)] \quad (3),$$

dans laquelle N est un entier qui représente le nombre de points utilisés pour effectuer la somme, $q_{d1}$ est la quantité de gaz donné dans les gaz extraits de l'échantillon d'analyse et b est le paramètre déterminé à l'étape de calibration.

En pratique, N est supérieur ou égal à 2, et de préférence compris entre 4 et 6.

Le nombre N est déterminé en fonction de l'aptitude du gaz donné à être extrait de la boue de forage dans la cuve 67B dans les conditions déterminées.

Ainsi, et comme illustré par la Figure 5, une famille 313, 315 distincte de courbes de calibration est obtenue pour chaque gaz donné. Sur la Figure 5, le premier gaz donné correspondant à la famille 313 représentée en haut de la Figure présente une cinétique d'extraction moins rapide que le deuxième gaz donné correspondant à la famille 315 du bas de la Figure. Un nombre de points $N_1$ plus élevé est donc choisi dans la formule (3) pour calculer la teneur en premier gaz donné par rapport au nombre de points $N_2$ choisi pour calculer la teneur en second gaz donné.

Par ailleurs, lorsqu'au moins deux gaz donnés sont quantifiés simultanément par l'instrumentation 101, la teneur relative en chacun des gaz donnés dans la boue de forage est déterminée par rapport à la teneur totale en gaz dans la boue.

En variante, un facteur de correction est appliqué pour déterminer de manière quantitative la teneur en un gaz donné dans la boue de forage.

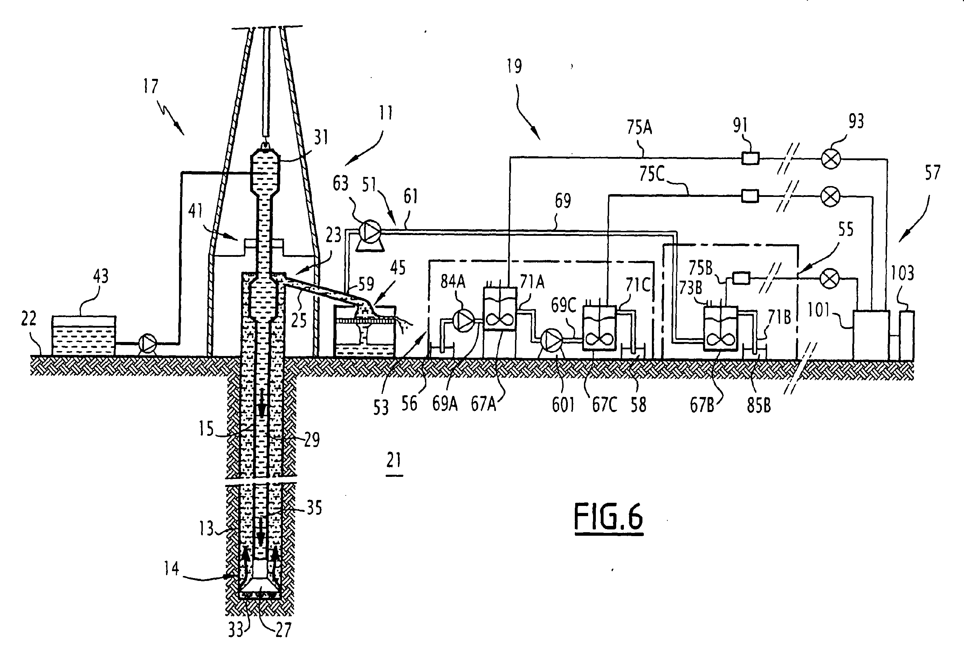

Dans un deuxième dispositif selon l'invention représenté en regard de la Figure 6, l'étage de calibration 53 comprend deux cuves 67A et 67C de structures identiques, montées en série. Ainsi, la conduite 71A d'évacuation de boue de la première cuve 67A est raccordée à la conduite 69C d'amenée de boue de la deuxième cuve 67C par une pompe 601. La conduite 71C d'extraction de boue de la deuxième cuve 67C est reliée au bac de rétention 58.

Lors de l'étape de calibration 201, le premier stade d'extraction 211 est effectué dans la première cuve 67A comme décrit précédemment. L'échantillon de calibration est alors récupéré et convoyé jusqu'à la deuxième cuve 67C où il subit le deuxième stade d'extraction 213.

En variante de ce deuxième dispositif, l'étage de calibration 53 peut comprendre une pluralité de cuves 67, montées en série.

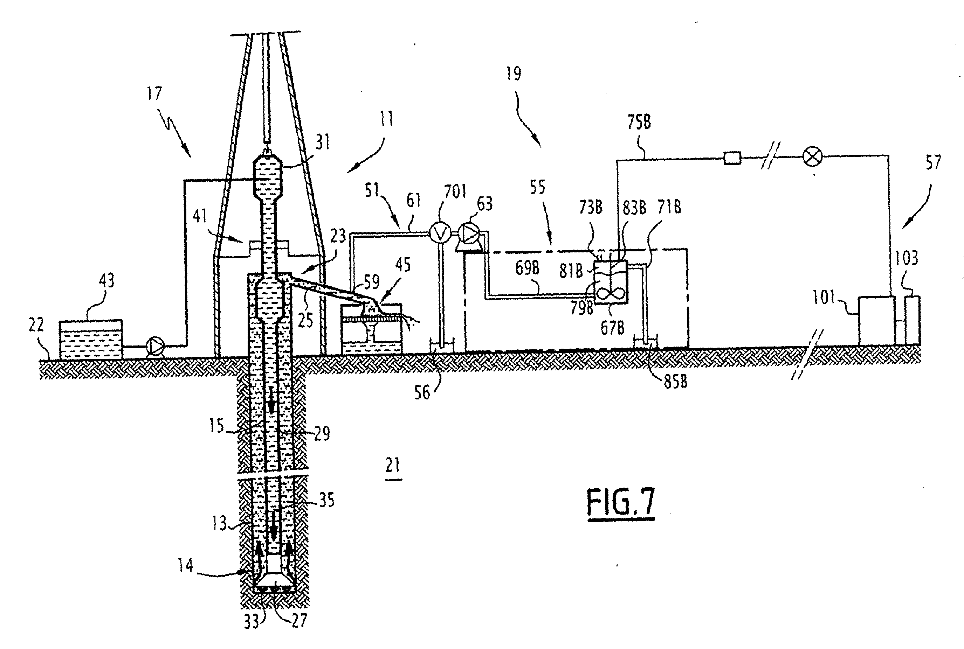

Dans un troisième dispositif selon l'invention représenté en regard de la Figure 7, l'étage de calibration 53 est constitué par l'étage d'analyse 55.

Une vanne trois voies 701 est interposée en amont de la pompe de prélèvement 63 sur la tubulure de raccordement 61. La vanne 701 comprend une entrée reliée au bac de réception 56 et une entrée reliée à la tête de prélèvement 59. Elle comprend une sortie raccordée à l'entrée de la pompe 63 sur la conduite d'amenée de boue 69B.

La vanne trois voies 701 est pilotée pour envoyer sélectivement à la cuve 67B la boue recueillie par les moyens de prélèvement 51 lors de l'étape d'analyse, ou la boue contenue dans le bac de réception 56 lors de l'étape de calibration. Le fonctionnement de ce dispositif est par ailleurs analogue à celui du premier dispositif.

Dans ce dispositif, l'étape de calibration 201 est réalisée par exemple à la fin d'une phase de forage. Durant la phase de forage, des échantillons d'analyse sont prélevés continûment et subissent chacun une phase de mesure 407. La phase de calcul 409A correspondant à chaque échantillon d'analyse est alors effectuée, à la fin de la phase de forage, après l'étape de calibration 201.

En variante, l'échantillon de calibration est obtenu à partir d'une boue de calibration distincte de la boue de forage.

Dans une autre variante, la famille de courbes 213 comprend une suite polynomiale ou une autre fonction mathématique.

Grâce à l'invention qui vient d'être décrite, il est possible de disposer d'un procédé qui permet la détermination de la teneur en au moins un gaz donné dans une boue de forage par des moyens simples. Ce procédé ne nécessite pas l'utilisation de modèles physico-chimiques complexes, notamment pour les boues à l'huile ou les boues d'origine synthétique.

**Revendications**

**1.**   Procédé de détermination de la teneur ($T_d$) en au moins un gaz donné dans une boue de forage, **caractérisé en ce qu'**il comprend :

   - une étape de calibration (201) comportant les phases de :

(i) mesurer (207) une information représentative de la quantité ($q_1$, $q_2$) de gaz donné dans les gaz extraits d'un échantillon de calibration d'une boue de calibration, à au moins deux stades (211, 213) d'extraction, dans des conditions déterminées, des gaz de l'échantillon de calibration ; et

(ii) établir (209) une famille (310) de courbes (303, 305, 309) représentatives de l'extraction, dans les conditions déterminées, du gaz donné contenu dans la boue de forage, sur la base des mesures effectuées lors des stades d'extraction (211, 213) des gaz de l'échantillon de calibration ; et

- au moins une étape d'analyse (203) comportant les phases de :

(a) mesurer (407) une information représentative de la quantité de gaz donné ($q_{d1}$) dans les gaz extraits d'un échantillon d'analyse de la boue de forage à un stade d'extraction (407), dans les conditions déterminées, des gaz de l'échantillon d'analyse ; et

(b) calculer (409A, 409B) la teneur ($T_d$) en gaz donné dans la boue de forage, sur la base de la quantité de gaz donné ($q_{d1}$) mesurée à l'étape (a) et d'une courbe (309) de ladite famille (310).

2. Procédé selon la revendication 1, **caractérisé en ce que**, lors de l'étape (i), chaque stade d'extraction (211, 213) comporte les étapes de :

- introduire (205) l'échantillon de calibration dans une première cuve d'extraction de gaz (67 A ; 67C) munie de moyens (83) d'agitation, à une température donnée ;
- prélever les gaz extraits dans la cuve (67A ; 67C) ;

et lors de l'étape (a), le stade d'extraction (407) comprend les étapes de:

- introduire l'échantillon d'analyse dans une deuxième cuve (67B) analogue à la première cuve (67A ; 67C), sensiblement à la température donnée,
- prélever les gaz extraits dans la deuxième cuve (67B).

3. Procédé selon la revendication 2, **caractérisé en ce que** lors de l'étape (i), chaque stade d'extraction (211, 213) comprend une étape de récupération (221) de l'échantillon de calibration après l'étape de prélèvement des gaz extraits.

4. Procédé selon l'une quelconque des revendications précédentes **caractérisé en ce que**, lors de l'étape (i), le nombre de stades d'extraction (211, 213, 215) est compris entre 2 et 6.

5. Procédé selon l'une quelconque des revendications précédentes, **caractérisé en ce que** ladite famille (310) de courbes (303, 305, 309) comprend au moins une fonction exponentielle.

6. Procédé selon la revendication 5, **caractérisé en ce que** la famille (310) de courbes comprend des suites (303, 305, 309) de formule générale :

$$q_n = q_1 \exp\left[- b\,(n-1)\right] \quad (1)$$

dans laquelle :

- n est le nième stade d'extraction de la phase d'extraction ;
- $q_n$ est la quantité de gaz donné dans les gaz extraits lors du nième stade d'extraction ;
- $q_1$ est la quantité de gaz donné dans les gaz extraits lors du premier stade d'extraction,
- b est un paramètre indépendant de la teneur en gaz donné dans la boue de calibration et qui dépend des caractéristiques de la boue et des conditions déterminées.

7. Procédé selon l'une quelconque des revendications précédentes, **caractérisé en ce que** la phase (b) comprend les étapes de :

- identifier la courbe (309) de ladite famille (310) sur laquelle se trouve un point (307) correspondant à la quantité de gaz donné ($q_{d1}$) mesurée à l'étape (a) et au stade d'extraction de l'échantillon d'analyse ; et

- effectuer la somme de cette quantité de gaz donné ($q_{d1}$) mesurée à l'étape (a) avec au moins une quantité de gaz donné ($q_{d2}$) correspondant à un autre point (311) de ladite courbe (309).

8. Procédé selon l'une quelconque des revendications précédentes, **caractérisé en ce que** la boue de forage est une boue à base d'huile ou une boue à base d'au moins un composé synthétique.

9. Procédé selon l'une quelconque des revendications précédentes, **caractérisé en ce que** la boue de calibration comprend au moins une partie de la boue de forage.

10. Dispositif (19) de détermination de la teneur ($T_d$) en au moins un gaz donné dans une boue de forage, du type comprenant :

    - des moyens (53) de calibration comportant :

        • des premiers moyens (67A, 83A) d'extraction, dans des conditions déterminées, du gaz contenu dans un échantillon de calibration d'une boue de calibration ;
        • des premiers moyens (101) de mesure d'une information représentative de la quantité ($q_1$, $q_2$) en au moins un gaz donné dans les gaz extraits de l'échantillon de calibration, à au moins deux stades d'extraction dans les conditions déterminées, dans les premiers moyens d'extraction (67A, 83A);
        • des moyens (103) d'établissement d'une famille (310) de courbes (303, 305, 309) représentatives de l'extraction, dans les conditions déterminées, du gaz donné contenu dans la boue de forage, sur la base des mesures effectuées par les premiers moyens de mesure (101) ;

    - des moyens d'analyse (55) comportant :

        • des deuxièmes moyens d'extraction (67B, 83B), dans les conditions déterminées, du gaz contenu dans un échantillon d'analyse de la boue de forage ;
        • des deuxièmes moyens (101) de mesure d'une information représentative de la quantité de gaz donné ($q_{d1}$) dans les gaz extraits de l'échantillon d'analyse, à un stade d'extraction dans les conditions déterminées, dans les deuxièmes moyens d'extraction (67B, 83B) ;
        • des moyens (103) de calcul de la teneur en gaz donné ($T_d$) dans la boue de forage, sur la base de la quantité de gaz donné ($q_{d1}$) mesurée par les deuxièmes moyens de mesure (101) et d'une courbe (309) de ladite famille (310).

11. Dispositif (19) selon la revendication 10, **caractérisé en ce que** les premiers et deuxièmes moyens d'extraction (67A, 83A ; 67B, 83B) comprennent chacun au moins une cuve (67A ; 67B) munie de moyens d'agitation (83A ; 83B), la ou chaque cuve (67A ; 67B) comportant :

    • des moyens (69A ; 69B) d'amenée de boue dans la cuve (67A ; 67B) ;
    • des moyens (71A; 71 B) d'évacuation de la boue hors de la cuve (67A ; 67B) ;
    • des moyens (75A ; 75B) de prélèvement du gaz extrait dans la cuve (67A ; 67B) ;

    lesdites cuves (67A , 67B) présentant des structures analogues.

12. Dispositif (19) selon la revendication 11, **caractérisé en ce que** les premiers moyens d'extraction (67A, 83A) comprennent une cuve unique (67A) et des moyens (58) de récupération de la boue à la sortie des moyens d'évacuation (71A), aptes à être raccordés à une entrée de la cuve (67A).

13. Dispositif (19) selon la revendication 12, **caractérisé en ce que** les premiers moyens d'extraction sont constitués par les deuxièmes moyens d'extraction (67B, 83B).

14. Dispositif (19) selon la revendication 11, **caractérisé en ce que** les premiers moyens d'extraction (67A, 83A ; 67C, 83C) comprennent au moins deux cuves (67A ; 67C) de structures analogues, les moyens (71A) d'évacuation de la boue de la première cuve (67A) étant raccordés aux moyens (69C) d'amenée de boue de la deuxième cuve (67C).

15. Installation de forage (11) du type comprenant un conduit (25) de circulation d'une boue de forage, **caractérisée en ce qu'**elle comprend :

- un dispositif (19) selon l'une des revendications 10 à 14 ; et
- des moyens (51) de prélèvement de boue dans le conduit de circulation (25).

**Claims**

1.  Method of determining the content ($T_d$) of at least one given gas in a drilling mud, **characterised in that** it comprises:

    - a calibration step (201) comprising the phases of:

        (i) measuring (207) a piece of information representative of the quantity ($q_1$, $q_2$) of given gas in the gases extracted from a calibration sample of a calibration mud, with at least two extraction stages (211, 213), under determined conditions, of the gases of the calibration sample; and
        (ii) establishing (209) a family (310) of curves (303, 305, 309) representative of the extraction, under determined conditions, of the given gas contained in the drilling mud, on the basis of the measurements carried out during the extraction stages (211, 213) of the gases of the calibration sample; and

    - at least one analysis step (203) comprising the phases of:

        (a) measuring (407) a piece of information representative of the quantity of given gas ($q_{d1}$) in the gases extracted from an analysis sample of the drilling mud at an extraction stage (407), under determined conditions, of the gases from the analysis sample; and
        (b) calculating (409A, 409B) the content ($T_d$) of given gas in the drilling mud, based on the quantity of given gas ($q_{d1}$) measured at step (a) and a curve (309) of the said family (310).

2.  Method according to claim 1, **characterised in that** during step (i), each extraction stage (211, 213) comprises the steps of:

    - introducing (205) the calibration sample into a first gas extraction tank (67A; 67C) equipped with means (83) of agitation, at a given temperature;
    - removing the extracted gases in the tank (67A; 67C);

    and during step (a), the extraction stage (407) comprises the steps of:

    - introducing the analysis sample into a second tank (67B) similar to the first tank (67A, 67C), approximately at the given temperature,
    - removing the extracted gases in the second tank (67B).

3.  Method according to claim 2, **characterised in that** during step (i), each extraction stage (211, 213) comprises a recovery step (221) of the calibration sample after the step of removing the extracted gases.

4.  Method according to any of the preceding claims, **characterised in that** during step (i), the number of extraction stages (211, 213, 215) is comprised between 2 and 6.

5.  Method according to any of the preceding claims, **characterised in that** the said family (310) of curves (303, 305, 309) comprise at least one exponential function.

6.  Method according to claim 5, **characterised in that** the family (310) of curves comprises series (303, 305, 309) of general formula:

$$q_n = q_1 \exp [- b (n - 1)] \quad (1)$$

in which:

    - n is the nth extraction stage of the extraction phase;
    - $q_n$ is the quantity of given gas in the extracted gases during the nth extraction stage;

- $q_1$ is the quantity of given gas in the extracted gases during the first extraction stage,
- b is a parameter independent of the content of given gas in the calibration mud and which is dependant on the characteristics of the mud and the determined conditions.

7. Method according to any of the preceding claims, **characterised in that** phase (b) comprises the steps of:

- identifying the curve (309) of the said family (310) on which there is a point (307) corresponding to the quantity of given gas ($q_{d1}$) measured at step (a) and to the extraction stage of the analysis sample; and
- carrying out the sum of this quantity of given gas ($q_{d1}$) measured at step (a) with at least one quantity of given gas ($q_{d2}$) corresponding to another point (311) of the said curve (309).

8. Method according to any of the preceding claims, **characterised in that** the drilling mud is a mud based on oil or a mud based on at least one synthetic compound.

9. Method according to any of the preceding claims, **characterised in that** the calibration mud comprises at least part of the drilling mud.

10. Device (19) for determining the content ($T_d$) of at least one given gas in a drilling mud, of the type comprising:

- means (53) of calibration containing:

• first means (67A), 83A) of extraction, under determined conditions, of the gas contained in a calibration sample of a calibration mud;
• first means (101) of measuring a piece of information representative of the quantity ($q_1$, $q_2$) of at least one given gas in the gases extracted from the calibration sample, at at least two extraction stages under the determined conditions, in the first extraction means (67A, 83A);
• means (103) of establishing a family (310) of curves (303, 305, 309) representative of the extraction, under the determined conditions, of the given gas contained in the drilling mud, on the basis of the measurements carried out by the first measurement means (101);

- means of analysis (55) comprising:

• second means of extraction (67B, 83B), under the determined conditions, of the gas contained in an analysis sample of the drilling mud;
• second means (101) of measuring a piece of information representative of the quantity of given gas ($q_{d1}$) in the gases extracted from the analysis sample, at an extraction stage under the determined conditions, in the second means of extraction (67B, 83B);
• means (103) of calculating the content of given gas ($T_d$) in the drilling mud, on the basis of the quantity of given gas ($q_{d1}$) measured by the second means of measurement (101) and of a curve (309) of the said family (310).

11. Device (19) according to claim 10, **characterised in that** the first and second means of extraction (67A, 83A; 67B, 83B) each comprise at least one tank (67A; 67B) equipped with means of agitation (83A; 83B), the or each tank (67A; 67B) comprising:

• means (69A; 69B) of conveying mud into the tank (67A; 67B);
• means (71A, 71 B) of evacuation of the mud outside the tank (67A; 67B);
• means (75A; 75B) of removing the gas extracted in the tank (67A; 67B); the said tanks (67A, 67B) presenting similar structures.

12. Device (19) according to claim 11, **characterised in that** the first means of extraction (67A, 83A) comprise a single tank (67A) and means (58) of recovering the mud at the outlet of the means of evacuation (71A), suitable for being connected to an inlet of the tank (67A).

13. Device (19) according to claim 12, **characterised in that** the first means of extraction are composed of the second means of extraction (67B, 83B).

14. Device (19) according to claim 11, **characterised in that** the first means of extraction (67A, 83A; 67C, 83C) comprise at least two tanks (67A; 67C) of similar structures, the means (71A) of evacuation of the mud from the first tank (67A) being connected to the means (69C) of conveying mud from the second tank (67C).

15. Drilling installation (11) of the type comprising a pipe (25) for circulating a drilling mud, **characterised in that** it comprises:

 - a device (19) according to one of claims 10 to 14; and
 - means (51) of removing mud in the circulating pipe (25).

**Patentansprüche**

1. Verfahren zur Bestimmung des Gehalts ($T_d$) an mindestens einem gegebenen Gas in einem Bohrschlamm, **dadurch gekennzeichnet, dass** es umfasst:

 - einen Kalibrierungsschritt (201), umfassend die Phasen:

 (i) Messen (207) einer Information, die für die Menge ($q_1$, $q_2$) von gegebenem Gas in den aus einer Kalibrierungsprobe eines Kalibrierungsschlamms extrahierten Gasen repräsentativ ist, in mindestens zwei Stadien (211, 213) der Extraktion der Gase der Kalibrierungsprobe unter bestimmten Bedingungen; und
 (ii) Erstellen (209) einer Familie (310) von Kurven (303, 305, 309), die repräsentativ sind für die Extraktion des in dem Bohrschlamm enthaltenen gegebenen Gases unter den bestimmten Bedingungen, auf der Basis der Messungen, die in den Stadien (211, 213) der Extraktion der Gase der Kalibrierungsprobe vorgenommen wurden; und

 - mindestens einen Analyseschritt (203), umfassend die Phasen:

 (a) Messen (407) einer Information, die für die Menge von gegebenem Gas ($q_{d1}$) in den aus einer Analyseprobe des Bohrschlamms extrahierten Gasen repräsentativ ist, in einem Stadium (407) der Extraktion der Gase der Analyseprobe unter den bestimmten Bedingungen; und
 (b) Berechnen (409A, 409B) des Gehalts ($T_d$) an gegebenem Gas in dem Bohrschlamm auf der Basis der im Schritt (a) gemessenen Menge von gegebenem Gas ($q_{d1}$) und einer Kurve (309) dieser Familie (310).

2. Verfahren nach Anspruch 1, **dadurch gekennzeichnet, dass** jedes Extraktionsstadium (211, 213) im Schritt (i) die Schritte umfasst:

 - Einführen (205) der Kalibrierungsprobe in einen ersten Gasextraktionsbehälter (67A; 67C), der mit Rührmitteln (83) versehen ist, mit einer gegebenen Temperatur;
 - Entnehmen der extrahierten Gase in dem Behälter (67A; 67C); und das Extraktionsstadium (407) im Schritt (a) die Schritte umfasst:
 - Einführen der Analyseprobe in einen dem ersten Behälter (67A; 67C) entsprechenden zweiten Behälter (67B) im Wesentlichen mit der gegebenen Temperatur,
 - Entnehmen der extrahierten Gase in dem zweiten Behälter (67B).

3. Verfahren nach Anspruch 2, **dadurch gekennzeichnet, dass** jedes Extraktionsstadium (211, 213) im Schritt (i) einen Schritt der Gewinnung (221) der Kalibrierungsprobe nach dem Schritt der Entnahme der extrahierten Gase umfasst.

4. Verfahren nach einem der vorhergehenden Ansprüche, **dadurch gekennzeichnet, dass** die Anzahl von Extraktionsstadien (211, 213, 215) im Schritt (i) zwischen 2 und 6 beträgt.

5. Verfahren nach einem der vorhergehenden Ansprüche, **dadurch gekennzeichnet, dass** die Familie (310) von Kurven (303, 305, 309) mindestens eine exponentielle Funktion umfasst.

6. Verfahren nach Anspruch 5, **dadurch gekennzeichnet, dass** die Familie (310) von Kurven Folgen (303, 305, 309) der allgemeinen Formel

$$q_n = q_1 \exp[-b(n-1)] \quad (1)$$

umfasst, in der:

- $n$ das n-te Extraktionsstadium der Extraktionsphase ist;
- $q_n$ die Menge von gegebenem Gas in den im n-ten Extraktionsstadium extrahierten Gasen ist;
- $q_1$ die Menge von gegebenem Gas in den im ersten Extraktionsstadium extrahierten Gasen ist,
- $b$ ein von dem Gehalt an gegebenem Gas in dem Kalibrierungsschlamm unabhängiger Parameter ist, der von den Merkmalen des Schlamms und von den bestimmten Bedingungen abhängt.

7. Verfahren nach einem der vorhergehenden Ansprüche, **dadurch gekennzeichnet, dass** die Phase (b) die Schritte umfasst:

- Identifizieren der Kurve (309) dieser Familie (310), auf der sich ein Punkt (307) befindet, der der im Schritt (a) gemessenen Menge von gegebenem Gas ($q_{d1}$) und dem Stadium der Extraktion der Analyseprobe entspricht; und
- Bilden der Summe dieser im Schritt (a) gemessenen Menge von gegebenem Gas ($q_{d1}$) mit mindestens einer Menge von gegebenem Gas ($q_{d2}$), die einem anderen Punkt (311) dieser Kurve (309) entspricht.

8. Verfahren nach einem der vorhergehenden Ansprüche, **dadurch gekennzeichnet, dass** der Bohrschlamm ein Schlamm auf Basis von Öl oder ein Schlamm auf Basis von mindestens einer synthetischen Verbindung ist.

9. Verfahren nach einem der vorhergehenden Ansprüche, **dadurch gekennzeichnet, dass** der Kalibrierungsschlamm mindestens einen Teil des Bohrschlamms umfasst.

10. Vorrichtung (19) zur Bestimmung des Gehalts ($T_d$) an mindestens einem gegebenem Gas in einem Bohrschlamm, umfassend:

- Kalibrierungsmittel (53), umfassend:

  • erste Mittel (67A, 83A) zur Extraktion des in einer Kalibrierungsprobe eines Kalibrierungsschlamms enthaltenen Gases unter bestimmten Bedingungen;
  • erste Mittel (101) zum Messen einer Information, die für die Menge ($q_1$, $q_2$) von mindestens einem gegebenen Gas in den aus der Kalibrierungsprobe extrahierten Gasen repräsentativ ist, in mindestens zwei Stadien der Extraktion unter den bestimmten Bedingungen in den ersten Extraktionsmitteln (67A, 83A);
  • Mittel (103) zum Erstellen einer Familie (310) von Kurven (303, 305, 309), die für die Extraktion des in dem Bohrschlamm enthaltenen gegebenen Gases unter den bestimmten Bedingungen repräsentativ ist, auf der Basis der von den ersten Messmitteln (101) vorgenommenen Messungen;

- Analysemittel (55), umfassend:

  • zweite Mittel (67B, 83B) zur Extraktion des in einer Analyseprobe des Bohrschlamms enthaltenen Gases unter den bestimmten Bedingungen;
  • zweite Mittel (101) zum Messen einer Information, die für die Menge von gegebenem Gas ($q_{d1}$) in den aus der Analyseprobe extrahierten Gasen repräsentativ ist, in einem Stadium der Extraktion unter den bestimmten Bedingungen in den zweiten Extraktionsmitteln (67B, 83B);
  • Mittel (103) zur Berechnung des Gehalts an gegebenem Gas ($T_d$) im Bohrschlamm auf der Basis der von den zweiten Messmitteln (101) gemessenen Menge von gegebenem Gas ($q_{d1}$) und einer Kurve (309) der Familie (310).

11. Vorrichtung (19) nach Anspruch 10, **dadurch gekennzeichnet, dass** die ersten und zweiten Extraktionsmittel (67A, 83A; 67B, 83B) jeweils mindestens einen Behälter (67A; 67B) umfassen, der mit Rührmitteln (83A; 83B) versehen ist, wobei der oder jeder Behälter (67A; 67B) umfasst:

  • Mittel (69A; 69B) zur Zufuhr des Schlamms in den Behälter (67A; 67B);
  • Mittel (71A; 71B) zur Abfuhr des Schlamms aus dem Behälter (67A; 67B);

• Mittel (75A; 75B) zur Entnahme des extrahierten Gases im Behälter (67A; 67B);

wobei die Behälter (67A, 67B) einander entsprechende Strukturen aufweisen.

12. Vorrichtung (19) nach Anspruch 11, **dadurch gekennzeichnet, dass** die ersten Extraktionsmittel (67A, 83A) einen einzigen Behälter (67A) und Mittel (58) zur Gewinnung des Schlamms am Austritt der Abfuhrmittel (71A) umfassen, die mit einem Eintritt des Behälters (67A) verbunden werden können.

13. Vorrichtung (19) nach Anspruch 12, **dadurch gekennzeichnet, dass** die ersten Extraktionsmittel aus den zweiten Extraktionsmitteln (67B, 83B) bestehen.

14. Vorrichtung (19) nach Anspruch 11, **dadurch gekennzeichnet, dass** die ersten Extraktionsmittel (67A, 83A; 67C, 83C) mindestens zwei Behälter (67A; 67C) mit einander entsprechenden Strukturen umfassen, wobei die Mittel (71A) zur Abfuhr des Schlamms aus dem ersten Behälter (67A) mit den Mitteln (69C) zur Schlammzufuhr des zweiten Behälters (67C) verbunden sind.

15. Bohranlage (11), umfassend eine Leitung (25) zum Umlauf eines Bohrschlamms, **dadurch gekennzeichnet, dass** sie umfasst:

- eine Vorrichtung (19) nach einem der Ansprüche 10 bis 14; und
- Mittel (51) zur Entnahme von Schlamm in der Umlaufleitung (25).

**FIG.1**

Prélèvement d'un échantillon de calibration de la boue de forage — 205

Première extraction des gaz contenus dans l'échantillon de calibration
217

Détermination de la quantité $q_1$ de gaz donné dans les gaz extraits au premier stade d'extraction et enregistrement de $q_1$ dans le calculateur
219

211

Récupération de l'échantillon de calibration après extraction
221

Deuxième extraction des gaz contenus dans l'échantillon de calibration
217

Détermination de la quantité $q_2$ de gaz donné dans les gaz extraits au deuxième stade d'extraction et enregistrement de $q_2$ dans le calculateur
219

213

Récupération de l'échantillon de calibration après extraction
221

207   201

• • •

nième extraction des gaz contenus dans l'échantillon de calibration

Détermination de la quantité $q_n$ de gaz donné dans les gaz extraits au nième stade d'extraction et enregistrement de $q_n$ dans le calculateur

215

Détermination d'un paramètre b pour définir une famille de suites du type : $q_n = q_1 \exp\left[-b(n-1)\right]$ à partir des valeurs $q_1$, $q_2$, ... $q_n$
209

## FIG.2

405 {
> Prélèvement d'un échantillon d'analyse de la boue de forage

Première extraction des gaz contenus dans l'échantillon d'analyse

411

407 {

Détermination de la quantité $q_{d1}$ de gaz donné dans les gaz extraits au premier stade d'extraction et enregistrement de $q_{d1}$ dans le calculateur

413

} 203

Calcul de la teneur $T_d$ en gaz donné dans la boue de forage par la formule :

409 {

$$T_d = \sum_{n=0}^{\infty} q_{d1} \cdot \exp\left[-b(n-1)\right] = \frac{q_{d1}}{\left[1 - \exp(-b)\right]}$$

409A

Prélèvement d'un échantillon d'analyse de la boue de forage

405

} 203

• • •

## FIG.3

FIG.4

FIG.5

FIG.6

FIG.7

EP 1 710 575 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2799790

- US 5859430 A